**Europäisches Patentamt**

**European Patent Office**

**Office européen des brevets**

(19)

(11) Publication number: **0 007 702**
**B1**

(12)

# EUROPEAN PATENT SPECIFICATION

(45) Date of publication of patent specification: **25.05.83**

(51) Int. Cl.³: **A 61 B 5/04, A 61 B 5/02**

(21) Application number: **79301191.7**

(22) Date of filing: **20.06.79**

(54) Apparatus for use in physiological monitoring.

(30) Priority: **21.06.78 US 917556**
**01.03.79 US 16427**

(43) Date of publication of application:
**06.02.80 Bulletin 80/3**

(45) Publication of the grant of the patent:
**25.05.83 Bulletin 83/21**

(84) Designated Contracting States:
**BE DE FR GB IT NL SE**

(56) References cited:
**DE - B - 2 133 304**
**FR - A - 2 162 777**
**GB - A - 1 316 072**
**US - A - 3 120 227**
**US - A - 3 800 784**
**US - A - 3 999 555**
**US - A - 4 011 875**

(73) Proprietor: **Neward, Theodore Chester**
**521 Scripps Drive**
**Claremont California (US)**

(72) Inventor: **Neward, Theodore Chester**
**521 Scripps Drive**
**Claremont California (US)**

(74) Representative: **Nettleton, John Victor**
**Abel & Imray Northumberland House 303-306**
**High Holborn**
**London, WC1V 7LH (GB)**

Courier Press, Leamington Spa, England.

## Apparatus for use in physiological monitoring

The present invention relates to apparatus for use in physiological monitoring suitable for attachment to a fetus during labor and delivery to monitor the fetal heart rate.

It is desirable to monitor the fetal heart rate continuously during labor and delivery in order to obtain an immediate indication of the presence of undue distress or danger to the fetus. Devices attached external to the mother's body have proven inadequate for this purpose because they do not clearly distinguish the fetal heart rate from that of the mother. Therefore, various devices have been developed to attachment directly to the fetus during labor.

An early device of this type consisted of a "C" shaped wire, insulated except at the opposed tips thereof, which was inserted into the uterus and attached to the fetal scalp manually or by a forcep. This device was difficult to manipulate and insert, and did not attach securely to the fetus. Later devices were developed that screwed into the skin, such as those disclosed in Hon et al. US—E—28,990 and Ruttgers US—A—3,750,650. Tachick US—A—3,472,234 discloses another screw-type electrode. These devices require a manual attachment technique that is unusual and difficult for a physician to perform.

Other intrauterine fetal monitoring devices have taken various forms. Egan US—A—3,326,207 discloses a pair of contact electrodes mounted on balloons. LaCroix US—A—3,850,242 discloses a barbed, fishhook-like electrode inserted by a forcep. Hunter, Jr. et al., US—A—3,120,227 and FR—A—2.162.777 disclose a spring steel electrode having forcep-like branches that converge sharply to a point. A cylindrical sleeve must remain in the uterus to engage the branches and hold them together, making this device cumbersome to work with.

In Neward US—A—3,989,038, there is disclosed a clip-like fetal electrode having opposed jaws normally held together by spring tension, mounted on a long tube which may be removed after the electrode is attached to the fetus. Attachment, however, is accomplished by manipulation of one of the jaws through a rachet and cam assembly in a manner which may be awkward for a physician to perform while the electrode is inserted into the uterus. An alternate means for inserting an electrode into a body cavity is disclosed in Kilpatrick US—A—3,087,486, consisting of a tubular carrier sleeve containing a plunger which is depressed to drive the electrode into the tissue, after which the sleeve may be withdrawn from the body. The insertion sleeve, however, is disclosed in connection with a cardiac electrode, and is therefore quite rigid since it must penetrate the chest wall. A similar rigid penetration tool is disclosed in Quinn US—A—3,416,534, but in connection with a helix-shaped cardiac electrode that is screwed into the myocardium and therefore has the same disadvantages as the screw-type electrodes described in the patents mentioned above. Another cardiac electrode in the form of a spiral is disclosed in Rasor et al., US—A—3,835,864, for intracardiac use as a Pacemaker. A transvenous or transarterial catheter is used for insertion.

The invention as claimed is intended to provide an improved apparatus for use in physiological monitoring.

The invention as claimed provides:

Apparatus for use in physiological monitoring comprising:

a clip-type electrode structure having a pair of resiliently biased legs, each of said legs supporting a clamping member, at least one of said members being of metal to serve as an electrode, and

means for connecting said metal electrode to monitoring equipment, characterised by the provision of:

a resilient web separating the pair of legs,

spring means acting upon said legs for urging said members together in a clamping fashion,

carrier means for housing said electrode structure prior to use with the clamping members separated, and

means for at least partially ejecting said electrode structure from said carrier means to allow said clamping members to be forced together and become attached to a subject when said carrier means is placed against such subject.

Preferably, said ejecting means comprises a pair of cam surfaces in the carrier means for engagement with mating surfaces of said legs.

Preferably, said ejecting means comprises an elongated tube connected to said carrier means, said tube having a plunger extending therethrough for exerting pressure against said electrode structure.

Such apparatus is particularly suitable for fetal monitoring. The assembly is easily inserted into the uterus during labor, is operated in a fashion familiar to doctors, attaches easily and safely to the fetus, requires little associated structure while attached, and may be easily detached from the fetus after delivery. Prior to attachment to the fetus the electrode structure is housed in the carrier with the clamping members held apart. When it is desired to attach the electrode structure to the fetus, the physician inserts the assembly into the uterus until the scalp of the fetus is encountered, and then activates the plunger much like a familiar hypodermic syringe to move the members of the electrode structure into engagement with the cam surfaces of the carrier. The electrode structure is then ejected from the carrier by the cam action, allowing the clamping portions thereof to come together by spring action to penetrate and grasp the fetal epidermis firmly. The carrier may then be removed from the uterus, leaving only the electrode and its associated wires which are then connected to conventional heart rate monitoring equipment.

Ways of carrying out the invention are described in detail below with reference to drawings which illustrate two specific embodiments, in which:—

Figure 1 is a perspective view of a portion of an electrode carrier according to an embodiment of the present invention, showing part of an electrode structure housed therein.

Figure 2 is a detailed side view, in cross section, of a portion of the electrode carrier of Figure 1, showing housed therein an electrode structure according to the present invention in partial cross section in a position suitable for storage prior to use.

Figure 3 is a side view in cross section of a portion of an electrode carrier as in Figure 2, showing the electrode structure as it is positioned after being partially ejected from the carrier for attachment to the fetal epidermis.

Figure 4 is a plan view of the end portion of a carrier according to the present invention opposite the end housing the electrode structure, prior to attachment of the electrode structure.

Figure 5 is a plan view as in Figure 4 illustrating the end portion of the carrier in readiness for attachment of the electrode structure.

Figure 6 is a side view, in partial cross section, of the end portion of the carrier shown in Figures 4 and 5.

Figure 7 is a perspective view of a portion of an electrode carrier according to a presently preferred embodiment of the present invention, showing part of an electrode structure housed therein.

Figure 8 is a detailed side view, in cross section, of a portion of the electrode carrier of Figure 7, showing housed therein an electrode structure according to the present invention in partial cross section in a position suitable for storage prior to use.

Figure 9 is another side cross sectional view of a portion of the electrode carrier of Figure 7, taken at right angles to the side view of Figure 8 and showing a portion of an electrode structure and a plunger housed therein as in Figure 8.

Figure 10 is an end cross-sectional view of the electrode carrier of Figure 7, showing detail of the internal shape thereof and the plunger housed therein.

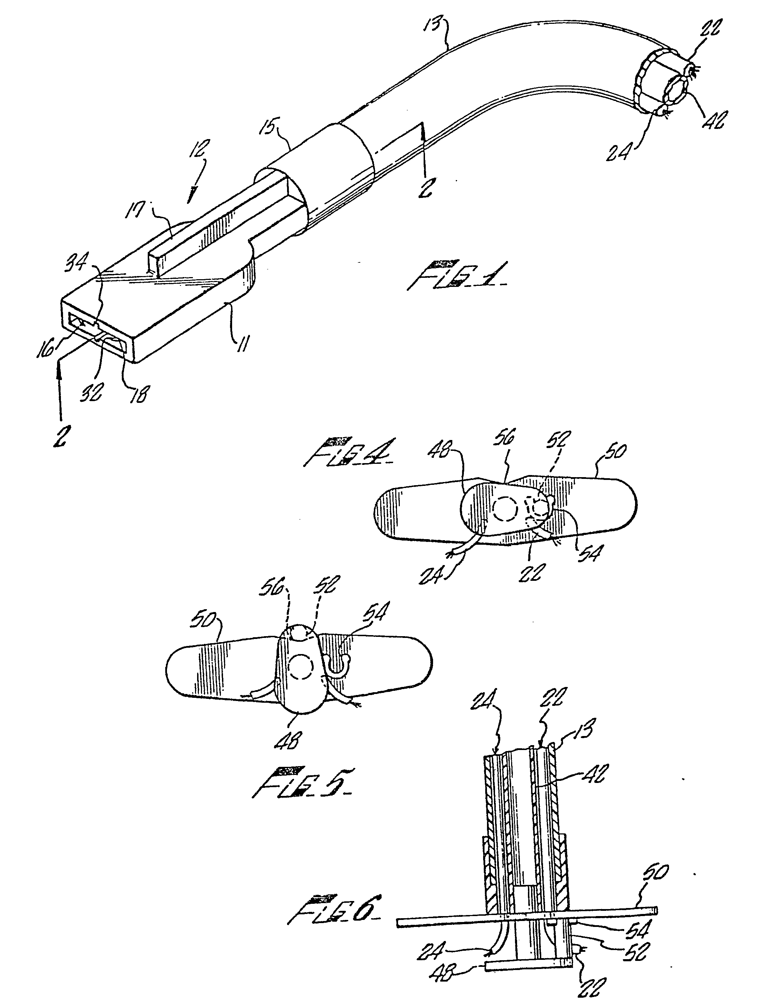

Referring first to Figure 1, there is illustrated a perspective view of an electrode carrier 12 according to one embodiment of the present invention. Within a cavity 32 of an electrode housing portion 11 of the carrier 12 there is housed an electrode structure to be described in greater detail hereinafter, the tips of whose clamping portions 16 and 18 may be viewed in Figure 1. The carrier 12 also includes a flexible or curved tube portion 13 extending from a cylindrical connecting member 15 and housing a plunger 40 having a flexible rod portion 42 shown in Figure 1 and wires 22 and 24, and the electrode housing portion 11 of the carrier 12 includes a raised portion 17 extending from both sides thereof for mating with the connecting member 15 and receiving a portion of the plunger 40, all to be described in greater detail hereinafter.

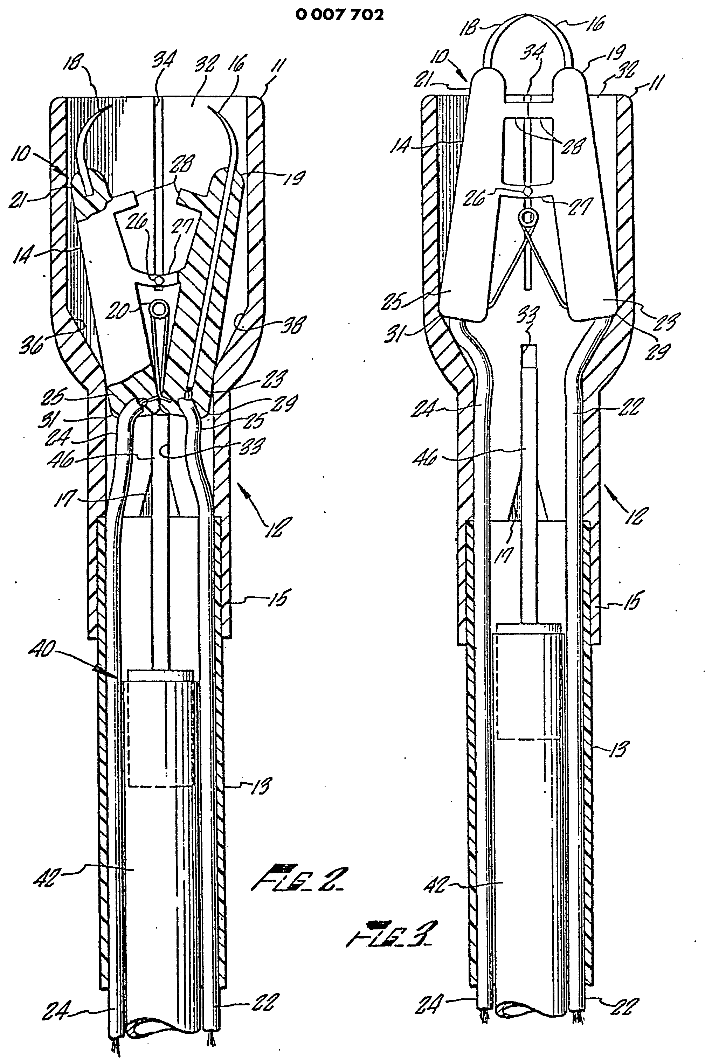

Referring next to Figure 2, which is a side view taken along the line 2—2 of Figure 1, there is illustrated an electrode structure 10 according to the present invention housed within the electrode carrier 12. The electrode structure 10 comprises a clip-type body 14 that may be constructed, for example, of a flexible plastic, having arms 19 and 21 supporting opposed metal clamping members 16 and 18, respectively, which are preferably metal wires sharpened to a point to easily penetrate the fetal epidermis, and further having legs 23 and 25 joined respectively to the arms 19 and 21, the pairs of arms and legs being connected by a resilient web 27 so that the body 14 possesses roughly the shape of the capital letter "H". A spring 20 which may be, for example, of a coil or compression type is embedded in the legs 23 and 25 of the body 14 to exert an outwardly expansive force on the legs, with the result that the clamping members 16 and 18 are normally urged together or toward the closed position. The member 16 illustrated in this embodiment serves also as a sensing electrode, and is connected through the body 14 to a wire 22 leading to a terminal (not shown) which may be connected to a monitoring unit. Another wire 24 leads from the body 14 and is connected within the body 14 to the spring 20, so that the spring 20 acts as a ground or reference electrode when the electrode structure 10 is surrounded by the maternal fluid present in the uterus prior to childbirth. A pair of guide pegs or pins 26 may be located on opposite sides of the web 27 of the body 14 for engagement with the carrier 12 as will be described in more detail hereinafter. Stops 28 may be provided on the facing surfaces of the arms 19 and 21 to allow the tips of the members 16 and 18 properly to engage each other and to limit the effective range of expansion of the spring 20.

The electrode carrier 12 includes an elongated flexible or curved cylinder or tube 13 and an electrode housing 11 joined by a cylindrical connecting member 15, as previously discussed in connection with Figure 1 and as also illustrated in Figure 2. The housing 11 and the connecting member 15 preferably comprise a single piece of molded plastic. The electrode housing 11 of the carrier 12 has an inner cavity 32 within which the electrode structure 10 is received. A raised portion 17 of the housing 11 provides a lower portion 33 of the cavity 32 within which the proximal end of the plunger 40 is received, as will be described in more detail hereinafter. Slots 34 may be formed in opposite walls of the cavity 32 to receive the guide pins 26 of the electrode structure 10 and thereby ensure proper alignment and simple attachment thereof. Cam surfaces 36 and 38 are formed in the lower part of the cavity 32, for engagement with mating surfaces 29 and 31 of the respective legs 23 and 25 of the electrode body 14, as will be described in greater detail hereinafter. The legs 23 and 25 are received within the connecting member 15 so that the clamping members 16 and 18 are separated while the electrode structure 10 is stored in the carrier 12.

Housed within the carrier 12 is a plunger 40 for moving the electrode structure 10 so that the mating surfaces 29 and 31 thereof move from their positions within the connecting member 15 onto the cam surfaces 36 and 38 for ejecting the electrode structure 10 from the carrier 12 and allowing the electrode structure 10 to attach to the subject. The plunger 40 comprises a flexible tube portion 42, part of which is shown in Figure 2, and an end portion 46 having a flat, rectangular end adjacent the electrode structure 10. The tube portion 42 is sufficiently smaller in diameter than the inside of the tube 13 to permit the electrode wires 22 and 24 to pass easily therethrough. The flat, rectangular end of the end portion 46 allows steady, even pressure to be exerted against the electrode structure 10 in a manner to be described in greater detail hereinafter. When the plunger 40 is forced against the electrode structure 10 in this manner, the end portion 46 thereof is received within the lower portion 33 of the cavity 32 formed in the raised portion 17 of the housing 11.

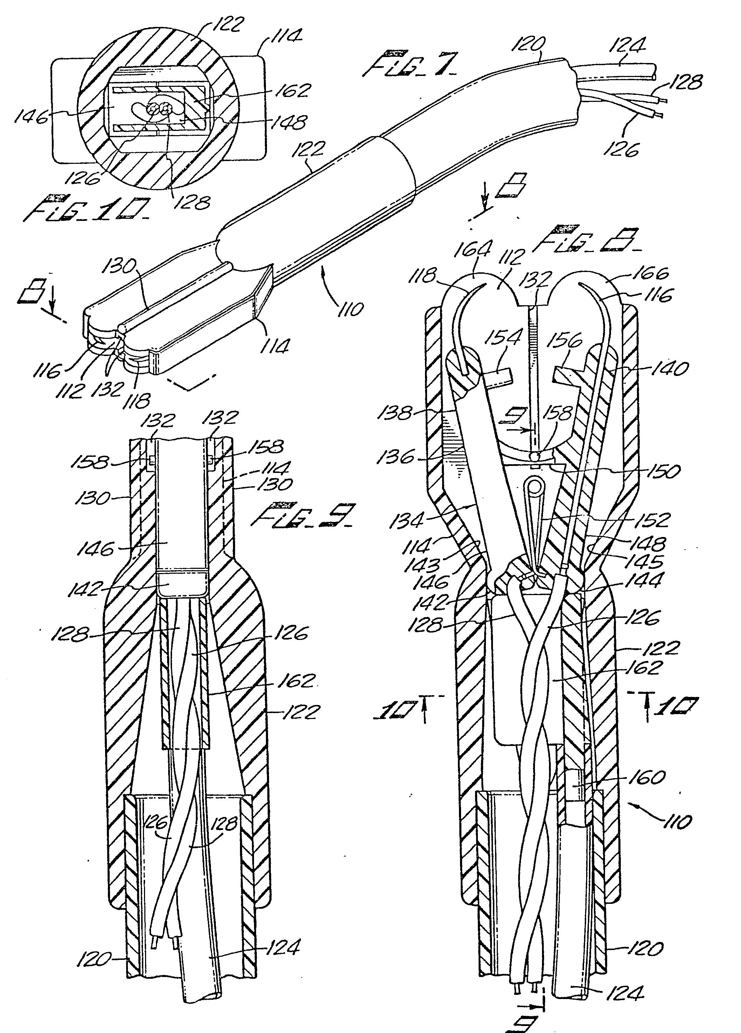

Referring to Figures 4, 5 and 6, at the end of the tube 13 opposite the housing 11 there is provided an actuator for operating the plunger 40. Into the end of the flexible tube portion 42 of the plunger 40 there is securely fitted a pressure plate 48. A base 50 is substantially parallel to the pressure plate 48 and is securely mated to the tube 13. For storage prior to use, a guard pin 52 affixed to the pressure plate 48 is inserted into a friction snap retainer or locator 54 in the base 50 as shown in Figures 4 and 6. A cutout 56 is provided in the base 50 for receiving the guard pin 52 when the plunger 40 is to be operated as shown in Figure 5. The pressure plate 48 is preferably asymmetrically shaped to permit ease of operation and certainty of positioning. The U-shaped design of the snap retainer 54 permits rotation of the pressure plate 48 only in the desired direction, as will be more fully explained below.

The electrode structure 10 is attached to the subject to be monitored in the following manner. The carrier 12 containing the recessed electrode structure 10 is positioned with the electrode housing 11 against the area of the skin surface to which the electrode structure 10 is to be attached. If the electrode structure 10 is to be attached to a fetus for heart rate monitoring during labor and delivery, for example, the carrier 12 is inserted into the uterus until the housing 11 contacts the head of the fetus. The physician prepares for attachment of the electrode structure 10 by rotating the plate 48 until the guard pin 52 is removed from the retainer 54, and then further rotating the plate 48 and the flexible tube portion 42 of the plunger 40 fitted thereto approximately 90° to a point where the guard pin 52 is received within the cutout 56 in the base 50. The flexible tube portion 42 of the plunger 40 possesses sufficient rotational flexibility to be rotatable approximately 90° while the end portion 46 of the plunger 40 adjacent the electrode structure 10 remains rotationally fixed within the lower portion 33 of the cavity 32 in the electrode housing 11. The physician then operates the plunger 40 in a manner substantially similar to the operation of a hypodermic syringe, by exerting thumb pressure on the pressure plate 48 and an opposite pressure with two fingers on the base 50. The flat rectangular end of the end portion 46 of the plunger 40 is thereby forced against the closed legs 23 and 25 of the electrode structure 10, causing the electrode structure 10 to be partially ejected from the housing 11. As the electrode structure 10 moves toward the open end of the housing 11, the mating surfaces 29 and 31 on the legs 23 and 25 engage the cam surfaces 36 and 38 of the housing 11, allowing the spring 20 to expand and exert outward force on the legs 23 and 25. The cam surfaces 36 and 38, aided by the expansive force of the spring 20 on the legs 23 and 25, then eject the electrode structure 10 on the legs 23 and 25, then eject the electrode structure 10 from the housing 11 to the position illustrated in Figure 3 without the application of further pressure by the plunger 40. For this purpose the cam surfaces 36 and 38 preferably comprise a self-lubricating material. In the position of Figure 3 the clamping members 16 and 18 are forced together by the spring 20, thus piercing and clamping the epidermis of the subject. Once the members 16 and 18 are clamped onto the subject, the carrier 12 may be removed completely, leaving only the electrode structure 10 with its associated wires 22 and 24. The distal ends of the wires 22 and 24 then may be connected to suitable monitoring equipment (not shown). When monitoring is no longer needed, as after delivery in the case of fetal monitoring, the electrode structure 10 is easily removed by manually compressing the legs 23 and 25 and thus forcing the clamping members 16 and 18 apart.

The body 14 of the electrode structure 10 according to the embodiment described above is preferably constructed by injection molding of a flexible plastic material of the same type used for the housing 11, described below. The clamping members 16 and 18 are approximately .01 inch (.254 mm) to .02 inch (.508 mm) in diameter and are preferably of polished stainless steel, as is the spring. The wires 22 and 24 are insulated copper wire of approximately 18 to 20 gauge.

The electrode structure 10 according to the preferred embodiment measures approximately 5/8" by 3/8" (1.59 cm × .95 cm) with the clamping members 16 and 18 protruding from the body approximately 1/8" (3.18 mm).

The housing 11 of the electrode carrier 12 is made of any suitable medically approved plastic of self-lubricating quality, and is preferably acetal resin but may be nylon or the like. The housing 11 is sized sufficiently larger than the electrode structure 10 to house the electrode structure 10 adequately. The tube portion 13 of the carrier 12 is made of a flexible or curved plastic or similar material such as linear polyethylene or nylon, and is approximately 11" (27.9 cm) long and 5/16" (7.94 mm) in diameter. The cylindrical connecting member 15 between the housing 11 and the tube 13 is part of the housing molding.

Figures 7—10 illustrate a presently preferred embodiment of the electrode and carrier assembly of the present invention. Referring first to Figure 7, there is illustrated in perspective a portion of an electrode carrier 110 similar to the electrode carrier 12 illustrated in Figure 1. Within a cavity 112 of an electrode housing portion 114 of the carrier 110 there is housed an electrode structure similar to that described above, the tips of whose clamping portions 116 and 118 are visible in Figure 7. The carrier 110 also includes a flexible or curved tube portion 120 extending from a cylindrical connecting member 122 and housing a tube 124 and twisted wires 126 and 128. Raised portions 130 on either side of the electrode housing 114 provide channels 132 within the cavity 112 for proper alignment of the electrode, in a manner to be described in more detail hereinafter.

Referring next to Figure 8, there is illustrated a side cross sectional view of the electrode carrier 110 of Figure 7, taken along the line 8—8 of Figure 7. An electrode structure designated generally as 134 is housed in the cavity 112 formed therein and is shown in partial cross section to illustrate its component parts. The general design of the electrode structure 134 is similar to that of the electrode structure 10 previously described. A clip-type body 136 includes arms 138 and 140 supporting opposed clamping members 118 and 116, and further includes legs 146 and 148 joined respectively to the arms 138 and 140, the pairs of arms and legs being connected by a resilient web 150 and the legs 146 and 148 having rounded surfaces 142 and 144 at their outer corners respectively for engaging cam surfaces 143 and 145 within the cavity 112. A spring 152 is embedded in the legs 146 and 148 to exert an outwardly expansive force thereon, urging the clamping members 118 and 116 into a closed position limited by stops 154 and 156. The wire 126 is connected through the leg 148 and the arm 140 to the clamping member 116, and the wire 128 is connected to the spring 152. Guide pins 158 are located on opposite sides of the web 150 for engagement with the slots 132 of the electrode housing 114. The functions performed by this electrode structure are substantially the same as those previously described in connection with the electrode structure 10.

Also illustrated in Figure 8 is the tube 124 received within the tube portion 120 of the electrode carrier 110. As illustrated in Figure 8, the tube 124 is preferably hollow for receiving a connecting pin 160 forming part of a U-shaped plunger 162 which is received within the cylindrical connecting portion 122 of the carrier assembly 110. The tube 124 may be connected to an actuating mechanism similar to that previously described in connection with Figures 4—6, whereby pressure may be exerted against the legs 146 and 148 of the electrode structure 134 to partially force the electrode structure 134 out of the carrier 110 in substantially the manner previously described, whereupon the carrier may be removed leaving only the electrode structure 134 and its associated wiring attached to the subject.

Two rounded protrusions 164 and 166 are molded into the proximal end of the electrode housing 114. When the housing 114 is pressed against the subject, such as the scalp of a fetus, these protrusions serve the purpose of gathering a portion of the fetal scalp into the space between the protrusions to provide an easier attachment to the fetal scalp by the clamping members 118 and 116. The depth of penetration of the clamping members into the fetal scalp is thereby more precisely controlled while still providing adequate electrical contact.

Detail of the interior of the electrode carrier 110 and the U-shaped plunger 162 received therein is illustrated in Figures 9 and 10, which will be referred to jointly. Figure 9 is a side cross sectional view taken along the line 9—9 of Figure 8, and Figure 10 is an end cross-sectional view taken along the line 10—10 of Figure 8. It will be observed that the interior of the cylindrical connecting portion 122 has a tapered shape for receiving the plunger 162 in the proper orientation. The twisted wires 126 and 128 are received within the U-shaped plunger 162 so that the plunger 162 encounters a minimum of drag or resistance from the wires 126 and 128 upon removal of the plunger 162 following attachment of the electrode structure 134 to the fetus. The tapered shape of the interior of the connecting portion 122 permits proper placement of the plunger 162 against the legs 146 and 148 to insure positive pressure for ejecting the electrode structure 134 from the housing 114. It may be desirable in some circumstances to utilize a single dual conductor wire in place of the two twisted wires to further reduce drag or resistance upon withdrawal of the plunger 162.

The design specifications and materials utilized in the preferred embodiment of Figures 7—10 are substantially the same as those previously described in connection with the embodiment of Figures 1—6.

## Claims

1. Apparatus for use in physiological monitoring comprising:

a clip-type electrode structure (10) having a pair of resiliently biased legs (23), each of said legs supporting a clamping member (16, 18), at least one of said members being of metal to serve as an electrode, and

means (22) for connecting said metal electrode to monitoring equipment, characterised by the provision of:

a resilient web (27) separating the pair of legs (23),

spring means (20) acting upon said legs for urging said members together in a clamping fashion,

carrier means (12) for housing said electrode structure prior to use with the clamping members separated, and

means (29, 31, 36, 38) for at least partially ejecting said electrode structure from said carrier means to allow said clamping members to be forced together and become attached to a subject when said carrier means is placed against such subject.

2. Apparatus as claimed in claim 1 wherein said ejecting means comprises a pair of cam surfaces (36, 38) in the carrier means for engagement with mating surfaces (29, 31) of said legs (23).

3. Apparatus as claimed in claim 1 or 2 wherein said ejecting means comprises an elongated tube (13) connected to said carrier means, said tube having a plunger (40) extending therethrough for exerting pressure against said electrode structure (10).

4. Apparatus as claimed in claims 2 and 3, wherein the elongated tube (13) has housing means (11) at one end thereof for receiving said electrode structure, and said housing means includes means for keeping said clamping members separated while said electrode structure is housed therein by engaging said legs and thereby compressing said tube for causing said mating surfaces to engage said cam surfaces.

5. Apparatus as claimed in claim 3 or 4, wherein the said elongated tube is a substantially cylindrical tube (120), the carrier means is a housing (114) at one end of said tube adapted to be placed against the skin of the subject, and means (164, 166) are mounted upon said housing means for gathering the skin of a subject when said housing is placed against the subject to facilitate secure attachment of said clamping members to the gathered skin when the electrode is ejected from the housing.

6. Apparatus as claimed in claim 5, wherein said means mounted upon said housing means for gathering the skin of the subject comprises a pair of protrusions (164, 166) extending from said housing means for gathering the skin of a subject therebetween.

7. Apparatus as claimed in claim 2 or 4 or any of claims 3, 5 and 6 as dependent on claim 2, wherein said electrode structure comprises a pair of opposed sharpened wires (116, 118) mounted upon arm members (138, 140), each arm member is connected to a respective one of the leg members (146, 148), said pairs of arm and leg members are joined by the resilient web (150), said leg members are connected by the spring (152) which urges said sharpened wires together, and said leg members have the said mating surfaces (142, 144) for engagement with said cam surfaces.

8. Apparatus as claimed in any preceding claim, wherein said electrode structure further comprises stop means (28; 154, 156) for limiting the range of expansion of said spring means and thereby limiting the degree of clamping of said clamping members.

## Revendications

1. Appareil destiné à être utilisé au cours d'un contrôle physiologique, comprenant:

une structure (10) à électrode du type à pince ayant une paire de branches (23) rappelées élastiquement, chaque branche supportant un organe de serrage (16, 18), l'un au moins de ces oranes étant formé d'un métal afin qu'il joue le rôle d'une électrode, et

un dispositif (22) de raccordement de l'électrode métallique à un équipement de contrôle, caractérisé par la présence:

d'une joue élastique (27) séparant les branches (23) de la paire,

d'un dispositif élastique (20) agissant sur les branches et destiné à repousser les organes l'un vers l'autre afin qu'ils soient serrés,

d'un dispositif (12) destiné à loger la structure à électrode avant l'utilisation alors que les organes de serrage sont séparés, et

d'un dispositif (29, 31, 36, 38) d'éjection au moins partielle de la structure à électrode hors du dispositif de support afin que les organes de serrage puisse être rapprochés et se fixent à un sujet lorsque le dispositif de support est placé contre un tel sujet.

2. Appareil selon la revendication 1, caractérisé en ce que le dispositif d'éjection comporte une paire de surfaces de came (36, 38) formées dans le dispositif de support et destinées à coopérer avec des surfaces complémentaires (29, 31) des branches (23).

3. Appareil selon l'une des revendications 1 et 2, caractérisé en ce que le dispositif d'éjection comporte un tube allongé (13) raccordé au dispositif de support, le tube ayant un piston (40) qui y est disposé à l'intérieur et qui est destiné à exercer une pression contre la structure à électrode (10).

4. Appareil selon l'une des revendications 2 et 3, caractérisé en ce que le tube allongé (13) comporte un dispositif de logement (11) à une première extrémité afin qu'il loge la structure à électrode, et le dispositif de logement comporte un dispositif destiné à maintenir séparés les organes de serrage pendant que la structure à électrode est logée à l'intérieur par coopération avec les branches et compression de cette manière du dispositif élastique, et le piston (40) passe dans le tube afin qu'il provoque la mise en contact des surfaces complémentaires et des surfaces de came.

5. Appareil selon l'une des revendications 3 et 4, caractérisé en ce que le tube allongé est un tube sensiblement cylindrique (120), le dispositif de support est un boîtier (114) placé à une première extrémité du tube et destiné à être placé contre la peau du sujet, et un dispositif (164, 166) est monté sur le dispositif de logement afin qu'il rapproche la peau d'un sujet lorsque le boîtier est placé contre le sujet et facilite la fixation ferme des organes de serrage à la peau resserrée lorsque l'électrode est éjectée du boîtier.

6. Appareil selon la revendication 5, caractérisé en ce que le dispositif monté sur le dispositif de logement pour le resserrage de la peau du sujet comporte une paire de saillies (164, 166) dépassant du dispositif de logement et destinées à resserrer entre elles la peau d'un sujet.

7. Appareil selon l'une des revendications 2 ou 4 ou selon l'une quelconque des revendications 3, 5 et 6 lorsqu'elle dépend de la revendication 2, caractérisé en ce que la structure à électrode comporte deux fils métalliques opposés et effilés (116, 118) montés sur des organes (138, 149) formant des bras et connectés chacun à un des organes formant des branches (146, 148), les paires d'organes formant des bras et des branches étant raccordées par la joue élastique (150), les organes formant les branches sont connectés par le ressort (152) qui rapproche les fils effilés, et les organes en forme de branches comportent les surfaces complémentaires (142, 144) destinées à coopérer avec les surfaces de came.

8. Appareil selon l'une quelconque des revendications précédentes, caractérisé en ce que la structure à électrode comporte en outre des butées (29; 154, 156) destinées à limiter la plage d'écartement du dispositif élastique et à limiter ainsi le legré de serrage des organes de serrage.

**Patentansprüche**

1. Vorrichtung zur Verwendung bei physiologischer Überwachung, umfassend:

eine klammerartige Eleketrodenstruktur (10) mit einem Paar von federnvorgespannten Schenkeln (23), wobei jeder der Schenkel ein Klammerglied (16, 18) abstützt und wobei mindestens eins der Glieder aus Metall besteht, um als Elektrode zu dienen und

Mittel (22) zum Verbinden der Metallelektrode mit einer Überwachungsanlage, gekennzeichnet durch das Vorsehen von:

Eines das Paar von Schenkeln (23) trennenden, nachgiebigen Zwischenstücks (27),

Fefermittel (20), die in Wirkverbindung mit den Schenkeln stehen für das gegeneinander Vorspannen der Glieder in klammerartiger Weise,

Trägermittel (12) zur Aufnahme der Elektrodenstruktur von ihrer Verwendung unter Trennung der Klammerglieder und

Mittel (29, 31, 36, 38) zum mindestens teilweisen Ausstoßen der Elektrodenstruktur aus den Trägermitteln, um den Klammergliedern das Zusammengepreßtwerden zu ermöglichen und zum Anheften an ein Subjekt, wenn die Trägermittel gegen ein solches Subjekt plaziert werden.

2. Vorrichtung nach Anspruch 1, worin die Ausstoßmittel ein Paar von Kulissenflächen (36, 38) in den Trägermitteln umfassen zum Zusammenwirken mit komplementären Flächen (29, 31) der Schenkel (23).

3. Vorrichtung nach Anspruch 1 oder 2, worin die Ausstoßmittel ein langgestrecktes Rohr (13) umfassen, angeschlossen an die Trägermittel, wobei das Rohr einen Schieber (40) aufweist, der sich hindurcherstreckt zum Ausüben von Druck gegen die Elektrodenstruktur (10).

4. Vorrichtung nach Anspruch 2 und 3, worin das langgestreckte Rohr (13) Gehäusemittel (11) an inem Ende desselben aufweist zur Aufnahme der Elektrodenstruktur und die Gehäusemittel Mittel umschließen zum Halten der Klammerglieder getrennt, während die Elektrodenstruktur darin aufgenommen ist, indem die Schenkel erfaßt werden und dabei die Federmittel komprimiert werden und worin der Schieber (40) sich durch das Rohr erstreckt zum Bewirken des Eingriffs der Komplementärflächen an den Kulissenflächen.

5. Vorrichtung nach Anspruch 3 oder 4, worin das langgestreckte Rohr ein im wesentlichen zylindrisches Rohr (120) ist, die Trägermittel ein Gehäuse (114) an einem Ende des Rohres sind, ausgebildet zum Plaziertwerden gegen die Haut des Subjekts, und Mittel (164, 166) an den Gehäusemitteln angebracht sind zum Erleichtern des sicheren Anhängens der des gehäuse gegen das Subjekt plaziert wird zum Erleichtern des sicheren Angängens der Klammerglieder an der erfaßten Haut, wenn die Elektrode aus dem Gehäuse ausgestoßen wird.

6. Vorrichtung nach Anspruch 5, worin die an dem Gehäuse angebrachten Mittel zum Erfassen der Haut des Subjektes ein Paar von Vorsprüngen (164, 166) umfassen, die sich von den Gehäusemitteln weg erstrecken zum Erfassen der Haut eines Subjektes zwischen ihnen.

7. Vorrichtung nach Anspruch 2 oder 4 oder einem der Ansprüche 3, 5 und 6, soweit sie von Anspruch 2 abhängen, worin die Elektrodenstruktur ein Paar von einander gegenüberstehenden geschärften Drähten (116, 118), montiert auf Armgliedern (138, 140) umfaßt, wobei jedes Armglied mit einem zugeordneten Schenkelglied (146, 148) verbunden ist, wobei die Paare von Armen und Schenkelgliedern durch das nachgiebige Zwischenstück (15) verbunden sind, wobei die Schenkelglieder durch die Feder (152) verbunden sind, welche die geschärften Drähte gegeneinander vorspannt und wobei die Schenkelglieder die Komplementärflächen (142, 144) zum Zusammenwirken mit den Kulissenflächen aufweisen.

8. Vorrichtung nach einem der vorangegehenden Ansprüche, worin die Elektrodenstruktur ferner Anschlagmittel (28, 154, 156) umfaßt zum Begrenzen des Bereiches der Expansion der Federmittel, um so den Klemmgrand der Klemmglieder zu begrenzen.

FIG.1.

FIG.4.

FIG.5.

FIG.6.

FIG 2.

FIG 3.

0 007 702

FiG_7.

FiG_10.

FiG_8.

FiG_9.

3